# EUROPEAN PATENT SPECIFICATION

(11) **EP 1 246 641 B1**
(45) Date of publication and mention of the grant of the patent: **11.08.2004**
(21) Application number: 01901984.3
(22) Date of filing: 12.01.2001
(51) Int. Cl.: A61K 38/46, A61P 31/04

(54) **BACTERIAL PHAGE ASSOCIATED LYSING ENZYMES FOR THE THERAPEUTIC TREATMENT OF BACTERIAL INFECTIONS**
BAKTERIOPHAGEN-ASSOZIIERTE LYTISCHE ENZYME ZUR THERAPEUTISCHEN BEHANDLUNG VON BAKTERIELLEN INFEKTIONEN
ENZYMES LYTIQUES ASSOCIES A UN PHAGE BACTERIEN DANS LE TRAITEMENT THERAPEUTIQUE DES INFECTIONS BACTERIENNES

(30) Priority: 14.01.2000 US 482992; 03.01.2001 US 752731
(43) Date of publication of application: 09.10.2002
(73) Proprietor: New Horizons Diagnostics, Corporation, Columbia, MD 21045-2014 (US)
(72) Inventor: FISCHETTI, Vincent, West Hempstead, NY 11552 (US); LOOMIS, Lawrence, Columbia, MD 21044 (US)
(74) Representative: Hinkelmann, Klaus, Dr.
(86) International application number: PCT/US2001/000913
(87) International publication number: WO 2001/051073

(56) References cited:
- EP-A- 0 510 907
- WO-A-96/07329
- WO-A-97/02351
- WO-A-99/04809
- US-A- 6 056 954
- US-A- 6 056 955
- DATABASE MEDLINE [Online] US NATIONAL LIBRARY OF MEDICINE (NLM), BETHESDA, MD, US; BABENKO IU S ET AL: "[Enzymatic lysis of staphylococci in relation to their species and strain properties]. Fermentativnyi lizis stafilokokkov v zavisimosti ot ikh vidovykh i shtammovykh osobennostei." retrieved from STN Database accession no. 90297672 XP002170795 & ANTIBIOTIKI I KHIMIOTERAPIIA, (1990 MAR) 35 (3) 20-2. ,

## Description

### BACKGROUND OF THE INVENTION

### 1. Field of the Invention

The present invention discloses a composition for the treatment of bacterial infections comprising lytic enzymes, holin lytic enzymes, shuffled lytic enzymes, and chimeric lytic enzymes, blended with an appropriate carrier into a patient. The injection can be done intramuscularly, subcutaneously, or intravenously.

### 2. Description of the Prior Art

In the past, antibiotics have been used to treat various infections. The work of Selman Waksman in the introduction and production of Streptomycetes, and Dr. Fleming 's discovery of penicillin, as well as the work of numerous others in the field of antibiotics, are well known. Over the years, there have been additions and chemical modifications to the "basic" antibiotics in attempts to make them more powerful, or to treat people allergic to these antibiotics.

Others have found new uses for these antibiotics. U.S. Patent No. 5,260,292 (Robinson et al.) discloses the topical treatment of acne with aminopenicillins. The method and composition for topically treating acne and acneiform dermal disorders includes applying an amount of an antibiotic, effective for treating acne and acneiform dermal disorders, selected from the group consisting of ampicillin, amoxicillin, other aminopenicillins, and cephalosporins, and derivatives and analogs thereof. U.S. Patent No. 5,409,917 (Robinson et al.) discloses the topical treatment of acne with cephalosporins.

However, as more antibiotics have been prescribed or used at an ever increasing rate for a variety of illnesses, increasing numbers of bacteria have developed a resistance to antibiotics. Larger doses of stronger antibiotics are now being used to treat ever more resistant strains of bacteria. Consequently, multiple antibiotic resistant bacteria have been developed. The use of more antibiotics and the number of bacteria showing resistance has led to increases in the amount of time that the antibiotics need to be used. Broad, non-specific antibiotics, some of which have detrimental effects on the patient, are now being used more frequently. Additionally, the number of people showing allergic reactions to antibiotics appears to be increasing.

Consequently, other efforts have been sought to first identify and then kill bacteria..

Attempts have been made to treat bacterial diseases with the use of bacteriophages. U.S. Patent No. 5,688,501 (Merril, et al.) discloses a method for treating an infectious disease caused by bacteria in an animal with lytic or non-lytic bacteriophages that are specific for particular bacteria.

U.S. Patent No. 4,957,686 (Norris) discloses a procedure of improved dental hygiene which introduces into the mouth bacteriophages parasitic to bacteria which possess the property of readily adhering to the salivary pellicle.

It is to be noted that the direct introduction of bacteriophages into an animal to prevent or fight diseases has certain drawbacks. Specifically, the bacteria must be in the right growth phase for the phage to attach. Both the bacteria and the phage have to be in the correct and synchronized growth cycles. Additionally, there must be the right number of phages to attach to the bacteria. The phage must also be active enough. The phages are also inhibited by many substances including bacterial debris from the organism it is going to attack. Further complicating the direct use of bacteriophage to treat bacterial infections is the possibility of immunological reactions, rendering the phage non-functional. Another problem is the mutation of the receptor on the bacterial surface preventing bacteriophage attachment.

Consequently, others have explored the use of other safer and more effective means to treat and prevent bacterial infections.

One bacteria for which a more effective treatment has been extensively explored is Streptococcus. The genus Streptococcus is comprised of a wide variety of both pathogenic and commensal gram-positive bacteria which are found to inhabit a wide range of hosts, including humans, horses, pigs, and cows. Within the host, streptococci are often found to colonize the mucosa surfaces of the mouth, nares and pharynx. However, in certain circumstances, they may also inhabit the skin, heart or muscle tissue.

Pathogenic streptococci of man include *S. pyogenes, S. pneumoniae,* and *S. faecalis.* While Group A streptococci may be present in the throat or on the skin and cause no symptoms of disease, they may also cause infections that range from mild to severe, and even life-threatening. Among the pathogenic hemolytic streptococci, *S. pyogenes,* or group A streptococci have been implicated as the etiologic agent of acute pharyngitis ("strep throat"), impetigo, rheumatic fever, scarlet fever, glomerulonephritis, and invasive fasciitis. Necrotizing fasciitis (sometimes described by the media as "the flesh-eating bacteria") is a destructive infection of muscle and fat tissue. Invasive group A streptococcal infections occur when the bacteria get past the defenses of the person who is infected. About 10,000-15,000 cases of invasive GAS disease occur in the United States each year, resulting in over 2,000 deaths. CDC estimates that 500 to 1,500 cases of necrotizing fasciitis and 2,000 to 3,000 cases of streptococcal toxic shock syndrome occur each year in the United States. Approximately 20% of patients with necrotizing fasciitis die, and 60% of patients with streptococcal toxic shock syndrome die. About 10 to 15% of patients with other forms of invasive group A streptococcal disease die.

Additionally, Group C Streptococcus can cause cellulitis from skin breaks, although cellulitis is normally associated with Staphylococcus aureus. Cellulitis can result in death, particularly in older individuals or in individuals who are already weakened.

Reports have described the characteristics of an enzyme produced by the group C streptococcal organism after being infected with a particular bacteriophage identified as Cl (Maxted, W.R. "The Active Agent in Nascent Page Lywsis of Streptococci," J. Gen Micro., vol 16, pp585-595, 1957, Krause, R.M., "Studies on the Bacteriophages of Hemolytic Streptococci," J. Exp. Med, vol. 108, pp 803-821, 1958) and Fischetti, (Fischetti, V.A., et al, "Purification and Physical Properties of Group C Streptococcal Phage Associated Lysin," J. Exp. Med, Vol 133 pp. 1105-1117, 1971) . The enzyme was given the name lysin and was found to specifically cleave the cell wall of group A, group C, and group E streptococci. These investigators provided information on the characteristics and activities of this enzyme with regard to lysing the group A streptococci and releasing the cell wall carbohydrate.

U.S. Patent No. 5,604,109 (Fischetti et al.) relates to the rapid detection of Group A streptococci in clinical specimens, through the enzymatic digestion by a semi-purified Group C streptococcal phage associated lysin enzyme. The present invention is based upon the discovery that phage lytic enzymes specific for bacteria infected with a specific phage can effectively and efficiently break down the cell wall of the bacterium in question. At the same time, the substrate for the enzyme is not present in mammalian tissues, and therefore is non-destructive to mammalian proteins and tissues when present during the digestion of the bacterial cell wall.

U.S. Patent No. 5,985,271 (Fischetti, et. al.), U.S. Patent No.5,997,862 (Fischetti et al.), and U.S. Patent No. 6,017,528 (Fischetti et al.) disclose the compositions and use of an oral delivery mode, such as a candy, chewing gum, lozenge, troche, tablet, a powder, an aerosol, a liquid or a liquid spray, containing a lysin enzyme produced by group C streptococcal bacteria infected with a C1 bacteriophage for the prophylactic and therapeutic treatment of Streptococcal A throat infections, commonly known as strep throat. This is the lysin enzyme of U.S. Patent No. 5,604,109

The same general technique used to produce and purify the lysin enzyme in U.S. Patent 5,604,109 may be used to manufacture other lytic enzymes produced by bacteria infected with a bacteriophage specific for that bacteria. Depending on the bacteria, there may be variations in the growth media and conditions.

The use of phage associated lytic enzymes produced by the infection of a bacteria with a bacteria specific phage has numerous advantages for the treatment of diseases. Lytic enzymes have similar characteristics as their complementary phage in that both are targeted for specific bacteria and neither, when selected, interferes with the functioning of normal bacterial flora.. Also, lytic phages primarily attack cell wall structures which are not affected by plasmid variation. The actions of the lytic enzymes are fast and do not depend on bacterial growth. Additionally, lytic enzymes can be directed to the mucosal lining, where, in residence, they will be able to kill colonizing bacteria.

U.S. Patent No. 6,056,954 (Fischetti et al.) discloses a method and composition for the prophylactic or therapeutic treatment of bacterial infections, comprising administering an effective amount of at least one lytic enzyme produced by a bacteria infected with a bacteriophage specific for the bacteria to the site of the infection. The lytic enzyme preferably comprises a carrier suitable for delivering the lytic enzyme to the site of the infection. This method and treatment may be used for treating upper respiratory infections, topical infections, vaginal infections, eye infections, ear infections, for parenteral treatment, and for most other bacterial infections.

U.S. Patent No. 6,056,955 (Fischetti et al.) discloses the topical treatment of streptococcal infections.

The use of phage associated lytic enzymes produced by the infection of a bacteria with a bacteria specific phage has numerous advantages for the treatment of diseases. As the phage are targeted for specific bacteria, the lytic enzymes do not interfere with normal flora. Also, lytic phages primarily attack cell wall structures which are not affected by plasmid variation. The actions of the lytic enzymes are fast and do not depend on bacterial growth.

However, sometimes the bacterial infections, by the time they are treated, have developed into more serious illnesses. For example, dermatological infections such as Staphylococcus aureus and Streptococcal pneumoniae can develop into cellulitis, which, unchecked, can lead to a degradation of the connective tissue, septicemia, and possibly death. Other bacterial infections can also evolve into deep tissue infections. Other infections by other bacteria, not necessarily dermatological by nature, can infect and localize in certain tissues of the body, making the infections difficult to treat.

U.S. Application No. 09/482,992 discloses a method and composition for the treatment of bacterial infections by the parenteral introduction of at least one lytic enzyme produced by a bacteria infected with a bacteriophage specific for that bacteria and an appropriate carrier for delivering the lytic enzyme into a patient. The injection can be done intramuscularly, subcutaneously, or intravenously.

### SUMMARY OF THE INVENTION

The present invention discloses the extraction and parenteral use of a variety of bacterial phage associated lytic enzymes, holin lytic enzymes, chimeric lytic enzymes, and shuffled lytic enzymes, in addition to lytic enzymes, for increased efficiency for the treatment of a wide variety of illnesses caused by bacterial infections. The lytic enzymes may be administered intravenously, subcutaneously, subdermally, or intramuscularly.

The method for obtaining and purifying the lytic enzyme produced by a bacteria infected with the bacteriophage is known in the art. Some recent evidence suggests that the phage enzyme that lyses the streptococcus organism may actually be a bacterial enzyme that is used to construct the cell wall and the phage. While replicating in the bacterium, a phage gene product may cause the upregulation or derepression of bacterial enzyme for the purpose of releasing the bacteriophage. These bacterial enzymes may be tightly regulated by the bacterial cell and are used by the bacteria for the construction and assembly of the cell wall.

The use of these lytic enzymes for the prophylactic and therapeutic treatment of bacterial diseases, however, has not been explored, except by the inventors of the present invention. The lytic enzymes produced by bacterial phages are specific and effective for killing select bacteria.

It is the current trend in biotechnology to do genomic sequencing of microorganisms in order, in part, to aid in the determination of designing drugs for treatment. This type of modeling is both timely and costly, and may or may not lead to new drug discoveries. The elegance of this invention resides in the fact that phage-induced lytic enzymes have evolved into extremely effective and targeted killing agents of selected bacteria. The primary structure and sequencing of purified enzymes serve as templates from which a variety of chemical procedures (such as shuffling) can be performed to optimize the enzyme's effectiveness.

These phage-induced lytic enzymes are useful in killing a variety of bacterial pathogens including those involved in classical clinical diseases such Streptococcus, Pseudomonas, etc. and in biowarfare agents, such as Bacillus and Yersinia, causing anthrax & plague, respectively, among other uses.

The invention uses an enzyme produced by the bacterial organism after being infected with a particular bacteriophage as a therapeutic treatment for those who have already become ill from the infection. The present invention is based upon the discovery that phage lytic enzymes specific for bacteria infected with a specific phage can effectively and efficiently break down the cell wall of the bacterium in question. At the same time, the semipurified enzyme is lacking in proteolytic enzymatic activity and therefore non-destructive to mammalian proteins and tissues when present during the digestion of the bacterial cell wall.

The creation, purification, and isolation of chimeric, shuffled, lytic, and holin enzymes are also well known to those skilled in the art. In particular, U.S. Patent No. 6,132,970 (Stemmer) discloses a number of new techniques, and modifications of more established procedures, for the creation of these enzymes. The proposed invention utilizes these techniques and applies them for the enhancement of specifically noted phage associated lytic enzymes. The technique for isolating lysin enzymes found in U.S. Patent No. 6,056,954, may be applied to other phage associated lytic enzymes. Similarly, other state of the art techniques may be used to isolate lytic enzymes.

For definitional purposes, shuffled enzymes are enzymes where more than one sequence of usually more than one particular enzyme has been cleaved in one or more locations, and reconstructed in a specific or random order, increasing their activity or specificity.

Chimeric enzymes are enzymes which are a combination of two or more enzymes having two or more active sites such that the chimeric enzyme can act independently on the same or different molecules. This will allow for potentially treating two or more different bacterial infections at the same time.

Holin enzymes produce holes in the cell membrane. More specifically, holin enzymes form lethal membrane lesions that terminate respiration. Like the lytic enzymes, the holin enzymes are coded for and carried by a phage genome. In fact, it is quite common for the genetic code for the holin enzyme to be found next to or even within the code for the lytic enzyme in the phage. Most holin sequences are short, and overall, hydrophobic in nature, with a highly hydrophilic carboxy-terminal domain. In many cases, the putative holin enzyme is encoded on a different reading frame within the enzymatically active domain of the phage. In other cases, the holin enzyme is encoded on the DNA next to or close to the DNA coding for the phage. The holin enzyme is frequently synthesized during the late stage of phage infection and found in the cytoplasmic membrane where it causes membrane lesions.

Holin enzymes can be grouped into two general classes based on primary structure analysis. Class I holins are usually 95 residues or longer and may have three potential transmembrane domains. Class II holins are usually smaller, at approximately 65-95 residues, and the distribution of charged and hydrophobic residues indicating two TM domains (Young, et al. *Trends in Microbiology* v. 8, No. 4, March 2000). At least for the phages of gram-positive hosts, however, the dual-component lysis system may not be universal. Although the presence of holins has been shown or suggested for several phages, no genes have yet been found encoding putative holins for all of the phages. Holins have been shown to be present or suggested for among others, lactococcal bacteriophage Tuc2009, lactococcal NLC3, pneumococcal bacteriophage EJ-1, *Lactobacillus gasseri* bacteriophage Nadh, *Staphylococcus aureus* bacteriophage Twort, *Listeria monocytogenes* bacteriophages, pneumococcal phage Cp-1, *Bacillus subtillis* phage M29, *Lactobacillus delbrueckki* bacteriophage LL-H lysin, and bacteriophage N11 of *Staphylococcus aureus*. (Loessner, et al., Journal of Bacteriology, Aug. 1999, p. 4452-4460).

The lytic system consists of a holin and at least one peptidoglycan hydrolase, or "lysin", capable of degrading the bacterial cell wall. Lysins can be endo--N-acetylglucosaminidases or N-acetylmuramidases (lysozymes), which act on the sugar moiety, endopeptidases which act on the peptide cross bridge, or more commonly, an N-acetylmuramoyl-L-alanine amidase, which hydrolyzes the amide bond connecting the sugar and peptide moieties. Typically, the holin is expressed in the late stages of phage infection forming a pore in the cell membrane, allowing the lysin(s) to gain access to the cell wall peptidoglycan resulting in release of progeny phage. Significantly, exogenously added lysin can lyse the cell wall of bacterial cells, producing a phenomenon known as "lysis from
without". However, in the case of gram-negative bacteria which contain an outer membrane, the presence of exogenously added holin in combination with a lytic enzyme may allow more efficient access of the lytic enzyme to the peptidoglycan enabling better lysis.

More specifically, the sequence of enzymes when purified can be determined by conventional techniques, and rearrangements of primary structures can be achieved by state of the art techniques, such as shuffling, to increase the activity and stability of the enzyme(s). Shuffling also allows for combination enzymes ("chimeric enzymes") to have more than one activity.

The use of phage associated lytic enzymes produced by the infection of a bacteria with a bacteria specific phage has numerous advantages for the treatment of diseases. As the phage are targeted for specific bacteria, the lytic enzymes do not interfere with normal flora. Also, lytic phages primarily attack cell wall structures which are not affected by plasmid variation. The actions of the lytic enzymes are fast and do not depend on bacterial growth.

The invention describes compositions comprising at least one phage associated lytic enzyme, at least one chimeric lytic enzyme, at least one shuffled lytic enzyme and at least one holin enzyme, which can be used, intravenously to treat septicemia, general infections, and deep tissue infections.

In one embodiment of the invention, the treatments of a variety of illnesses caused by *Streptococcus fasciae, and Staphylococcus aureus* are disclosed.

In yet another embodiment of the invention, lysostaphin, the enzyme which lyses Staphylococcus aureus, can be included in the therapeutic agent.

In a further embodiment of the invention, conventional antibiotics may be included in the therapeutic agent with the lytic enzyme, and with or without the presence of lysostaphin.

In another embodiment of the invention, at least one lytic enzyme, at least one chimeric lytic enzyme, at least one shuffled lytic enzyme, and at least one holin enzyme, are included in the therapeutic agent.

The therapeutic agent may be given parenterally, by means of an intramuscular, intradermal, or subcutaneous injection, or the agent may be given intravenously.

It is another object of the invention to provide compositions comprising at least one phage associated lytic enzyme, at at least one chimeric lytic enzyme, at least one shuffled lytic enzyme and at least one holin enzyme for intravenous administration to treat septicemia and general infections.

In one embodiment of the invention, the treatments of a variety of illnesses caused by *Streptococcal pneumoniae, Streptococcus fasciae, and Staphylococcus aureus* are disclosed.

In yet another embodiment of the invention, lysostaphin, the enzyme which lyses Staphylococcus aureus, can be included in the therapeutic agent.

In a further embodiment of the invention, conventional antibiotics may be included in the therapeutic agent with or without the presence of lysostaphin.

In a preferred embodiment of the invention, at least one lytic enzyme, at least one chimeric lytic enzyme, at least one shuffled lytic enzyme and at least one holin enzyme can be used to treat bacterial infections, thereby increasing the speed and efficiency with which the bacteria are killed.

Chimeric enzymes may also be used to treat one bacterial infection by cleaving the cell wall in more than one location.

A number of chimeric lytic enzymes have been produced and studied. Gene E-L, a chimeric lysin constructed from bacteriophages phi X174 and MS2 lysis proteins E and L, respectively, was subjected to internal deletions to create a series of new E-L clones with altered lysis or killing properties. The lytic activities of the parental genes E, L, E-L, and the internal truncated forms of E-L were investigated in this study to characterize the different lysis mechanism, based on differences in the architecture of the different membranes spanning domains. Electron microscopy and release of marker enzymes for the cytoplasmic and periplasmic spaces revealed that two different lysis mechanisms can be distinguished depending on penetrating of the proteins of either the inner membrane or the inner and outer membranes of the E. coli. FEMS Microbiol. Lett. 1998 Jul 1, 164(1); 159-67.

In another experiment an active chimeric cell wall lytic enzyme (TSL) has been constructed by fusing the region coding for the N-terminal half of the lactococcal phage Tuc2009 lysin and the region coding for the C-terminal domain of the major pneumococcal autolysin. The chimeric enzyme exhibited a glycosidase activity capable of hydrolysing choline-containing pneumococcal cell walls.

A preferred embodiment of this invention discloses the use of chimeric lytic enzymes to treat two infectious bacteria at the same time, or to cleave the cell wall of a bacteria in two different locations.

Holin enzymes are used in conjunction with the lytic enzymes to accelerate the speed and efficiency at which the bacteria are killed. Holin enzymes may also be in the form of chimeric and/or shuffled enzymes.

In another embodiment of the invention, the holin enzymes are shuffled holin enzymes or chimeric holin enzymes.

### BRIEF DESCRIPTION OF THE DRAWING

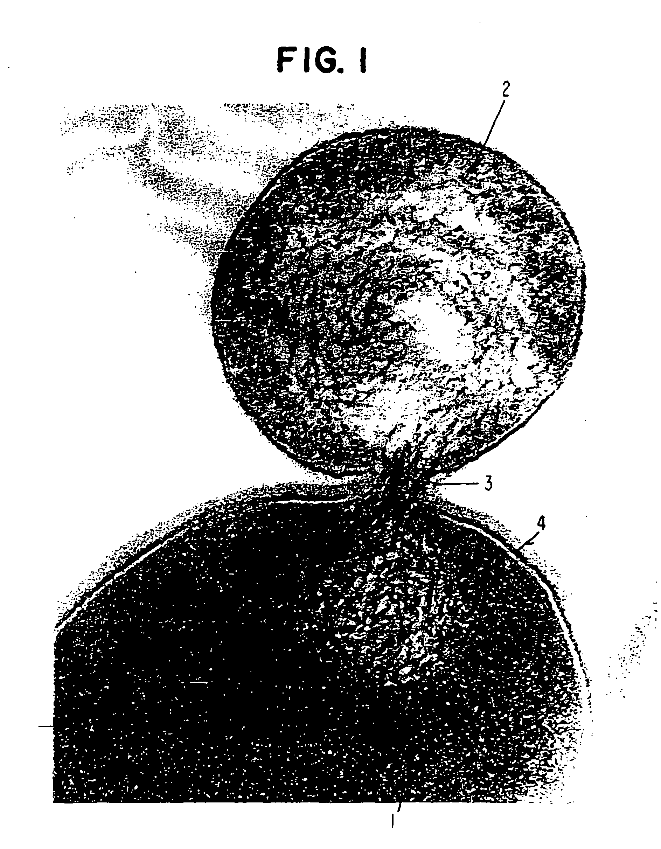

**Fig. 1** is an electron micrograph of group A streptococci treated with lysin showing the formation of a hole in the cell wall causing the cytoplasmic membrane to extrude and the cell contents to pour out.

### DETAILED DESCRIPTION OF THE INVENTION

Described is a method for treating systemic or tissue bacterial infections which comprises parenterally treating the infection with a therapeutic agent comprising an effective amount of at least one lytic enzyme, at least one chimeric lytic enzyme, at least one shuffled lytic enzyme, produced by a bacteria infected with a bacteriophage specific for the bacteria a least one holin enzyme, and an appropriate carrier.

The method described for treating bacterial infections comprises treating the infection with a therapeutic agent comprising an effective amount of a modified version of lytic enzymes produced by a bacteria infected with a bacteriophage specific for the bacteria wherein the modified version of the lytic enzyme is lytic enzymes, shuffled lytic enzymes and chimeric lytic enzymes. The lytic enzyme is preferably in an environment having a pH which allows for activity of said lytic enzyme. A holin enzyme is used in conjunction with the administration of the modified lytic enzymes. The holin enzyme may be in its "natural" state, and may be a shuffled holin enzyme or may be a chimeric lytic enzyme.

The shuffled and chimeric lytic enzymes may be produced either enzymatically or through recombinant DNA means. Any method may be used to produce these enzymes. The shuffled and chimeric lytic enzymes may be referred to as modified or engineered lytic enzymes.

The lytic enzyme can be used for the treatment or prevention of *Hemophilus influenza, Pseudomonas, Streptococcus pneumoniae*, *Streptococcus fasciae, Streptococcus* group B, *Listeria, Salmonella, E. coli, Campylobacter,* and other bacteria, and any combination thereof. This lytic enzyme may be either supplemented by chimeric and/or shuffled lytic enzymes, or may be itself a chimeric and/or shuffled lytic enzyme. Similarly, a holin enzyme is included, which may also be a chimeric and/or shuffled lytic enzyme.

A number of different bacteria may be treated. Among the bacteria which most often infect deep tissues, and, more specifically connective tissues, are Group A Streptococcus, Staphylococcus, Pseudomonas, and Clostridium. More than one lytic enzyme, and more than holin lytic enzyme, shuffled lytic enzyme, chimeric lytic enzyme, or combinations thereof, may be introduced into the infected body at a time.

A number of different methods may be used to introduce the lytic enzyme(s). These methods include introducing the modified lytic enzyme intravenously, intramuscularly, subcutaneously, and subdermally.

A deep tissue infection may be treated by injecting into the infected tissue of the patient a therapeutic agent comprising the appropriate holin lytic enzyme, chimeric lytic enzyme, shuffled lytic enzyme(s), lytic enzyme and a carrier for the enzyme. The lytic enzymes used may be either supplemented by chimeric and/or shuffled lytic enzymes, or may be itself a chimeric and/or shuffled lytic enzyme. Similarly, a holin enzyme is included, which may also be a chimeric and/or shuffled lytic enzyme. The carrier may be comprised of distilled water, a saline solution, albumin, a serum, or any combinations thereof. More specifically, solutions for infusion or injection may be prepared in a conventional manner, e.g. with the addition of preservatives such as p-hydroxybenzoates or stabilizers such as alkali metal salts of ethylene-diamine tetraacetic acid, which may then be transferred into fusion vessels, injection vials or ampules. Alternatively, the compound for injection may be lyophilized either with or without the other ingredients and be solubilized in a buffered solution or distilled water, as appropriate, at the time of use. Non-aqueous vehicles such as fixed oils and ethyl oleate are also useful herein. In cases where intramuscular injection is the chosen mode of administration, an isotonic formulation is preferably used. Generally, additives for isotonicity can include sodium chloride, dextrose, mannitol, sorbitol and lactose. In some cases, isotonic solutions such as phosphate buffered saline are preferred. Stabilizers include gelatin and albumin. In some embodiments, a vasoconstriction agent is added to the formulation. The pharmaceutical preparations according to the present invention are provided sterile and pyrogen free.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; glycine; amino acids such as glutamic acid, aspartic acid, histidine, or arginine; monosaccharides, disaccharides, and other carbohydrates including cellulose or its derivatives, glucose, mannose, trehalose, or dextrins; chelating agents such as EDTA; sugar alcohols such as mannitol or sorbitol; counter-ions such as sodium; non-ionic surfactants such as polysorbates, poloxamers, or polyethylene glycol (PEG); and/or neutral salts, e.g., NaCl, KCl, MgCl.sub.2, CaCl.sub.2, etc. Glycerin or glycerol (1,2,3-propanetriol) is commercially available for pharmaceutical use. It may be diluted in sterile water for injection, or sodium chloride injection, or other pharmaceutically acceptable aqueous injection fluid, and used in concentrations of 0.1 to 100% (v/v), preferably 1.0 to 50% more preferably about 20%. DMSO, which is an aprotic solvent with a remarkable ability to enhance penetration of many locally applied drugs. DMSO may be diluted in sterile water for injection, or sodium chloride injection, or other pharmaceutically acceptable aqueous injection fluid, and used in concentrations of 0.1 to 100% (v/v).

The carrier vehicle may also include Ringer's solution, a buffered solution, and dextrose solution, particularly when an intravenous solution is prepared..

Prior to, or at the time the lytic enzymes, including the modified lytic enzymes, are put in the carrier system or oral delivery mode, it is preferred that the enzymes be in a stabilizing buffer environment for maintaining a pH range between about 4.0 and about 9.0, more preferably between about 5.5 and about 7.5 and most preferably at about 6.1. This is pH range is most suitable for the lysin enzyme for Streptococcus.

The stabilizing buffer should allow for the optimum activity of the modified lytic enzyme. The buffer may be a reducing reagent, such as dithiothreitol. The stabilizing buffer may also be or include a metal chelating reagent, such as ethylenediaminetetracetic acid disodium salt, or it may also contain a phosphate or citrate-phosphate buffer. The buffers found in the carrier can serve to stabilize the environment for the modified lytic enzymes.

The effective dosage rates or amounts of the modified lytic enzymes to treat the infection, and the duration of treatment will depend in part on the seriousness of the infection, the duration of exposure of the recipient to the infectious bacteria, the number of square centimeters of skin or tissue which are infected, the depth of the infection, the seriousness of the infection, and a variety of a number of other variables. The composition may be applied anywhere from once to several times a day, and may be applied for a short or long term period. The usage may last for days or weeks. Any dosage form employed should provide for a minimum number of units for a minimum amount of time. The concentration of the active units of enzyme believed to provide for an effective amount or dosage of enzyme may be in the range of about 100 units/ml to about 500,000 units/ml of composition, preferably in the range of about 1000 units/ml to about 100,000 units/ml, and most preferably from about 10,000 to 100,000 units/ml. The amount of active units per ml and the duration of time of exposure depends on the nature of infection, and the amount of contact the carrier allows the modified lytic enzyme(s) to have. It is to be remembered that the modified lytic enzyme(s) works best when in a fluid environment. Hence, effectiveness of the modified lytic enzymes(s) in part related to the amount of moisture trapped by the carrier. For the treatment of septicemia, there should be a continuous intravenous flow of therapeutic agent into the blood stream. The concentration of modified lytic enzymes for the treatment of septicemia is dependent upon the seriousness of the infection.

In order to accelerate treatment of the infection, the therapeutic agent may further include at least one complementary agent which can also potentiate the bactericidal activity of the modified lytic enzymes. The complementary agent can be penicillin, synthetic penicillins bacitracin, methicillin, cephalosporin, polymyxin, cefaclor. Cefadroxil, cefamandole nafate, cefazolin, cefixime, cefinetazole, cefonioid, cefoperazone, ceforanide, cefotanme, cefotaxime, cefotetan, cefoxitin, cefpodoxime proxetil, ceftazidime, ceftizoxime, ceftriaxone, cefriaxone moxalactam , cefuroxime, cephalexin, cephalosporin C, cephalosporin C sodium salt, cephalothin, cephalothin sodium salt, cephapirin, cephradine, cefuroximeaxetil, dihydratecephalothin, moxalactam, loracarbef. mafate, chelating agents and any combinations thereof in amounts which are effective to synergistically enhance the therapeutic effect of the modified lytic enzyme(s).

Additionally, the therapeutic agent may further comprise the enzyme lysostaphin for the treatment of any *Staphylococcus aureus* bacteria. Mucolytic peptides, such as lysostaphin, have been suggested to be efficacious in the treatment of *S. aureus* infections of humans (Schaffner et al., Yale J. Biol. & Med., 39:230 (1967) and bovine mastitis caused by *S. aureus* (Sears et al., J. Dairy Science, 71 (Suppl. 1): 244(1988)). Lysostaphin, a gene product of *Staphylococcus simulans,* exerts a bacteriostatic and bactericidal effect upon *S. aureus* by enzymatically degrading the polyglycine cross-link of the cell wall (Browder et al., Res. Comm., 19: 393-400 (1965)). U.S. Pat. No. 3,278,378 describes fermentation methods for producing lysostaphin from culture media of *S. staphylolyticus,* later renamed *S. simulans.* Other methods for producing lysostaphin are further described in U.S. Pat. Nos. 3,398,056 and 3,594,284. The gene for lysostaphin has subsequently been cloned and sequenced (Recsei et al., Proc. Natl. Acad. Sci. USA, 84: 1127-1131 (1987)). The recombinant mucolytic bactericidal protein, such as r-lysostaphin, can potentially circumvent problems associated with current antibiotic therapy because of its targeted specificity, low toxicity and possible reduction of biologically active residues. Furthermore, lysostaphin is also active against non-dividing cells, while most antibiotics require actively dividing cells to mediate their effects (Dixon et al., Yale J. Biology and Medicine, 41: 62-68 (1968)). Lysostaphin, in combination with the modified lytic enzyme(s) can be used in the presence or absence of the listed antibiotics. There is a degree of added importance in using both lysostaphin and the lysin enzyme in the same therapeutic agent. Frequently, when a body has a bacterial infection, the infection by one genus of bacteria weakens the body or changes the bacterial flora of the body, allowing other potentially pathogenic bacteria to infect the body. One of the bacteria that sometimes co-infects a body is *Staphylococcus aureus.* Many strains of *Staphylococcus aureus* produce penicillinase, such that *Staphylococcus, Streptococcus,* and other gram positive bacterial strains will not be killed by standard antibiotics. Consequently, the use of the modified lytic enzyme(s) and lysostaphin, possibly in combination with antibiotics, can serve as the most rapid and effective treatment of bacterial infections. In yet another preferred embodiment, the invention may include mutanolysin, and lysozyme.

The use of lytic enzymes, including but not limited to holin lytic enzymes, chimeric lytic enzymes, shuffled lytic enzymes, and combinations thereof, rapidly lyse the bacterial cell. The thin section electron micrograph of Figure 1 shows the results of a group A streptococci 1 treated for15 seconds with lysin. The micrograph (25,000X magnification) shows the cell contents 2 pouring out through a hole 3 created in the cell wall 4 by the lysin enzyme.

As noted above, the use of the holin lytic enzyme, the chimeric lytic enzyme, and the shuffled lytic enzyme, may be accompanied by the use of a "natural" lytic enzyme, which has not been modified by the methods cited in U.S. Patent No. 6,132,970, or by similar state of the art methods.

Many modifications and variations of the present invention are possible in light of the above teachings. It is, therefore, to be understood within the scope of the appended claims the invention may be protected otherwise than as specifically described.

## Claims

1. Therapeutic agent comprising an effective amount of at least one lytic enzyme, at least one chimeric lytic enzyme and at least one shuffled lytic enzyme produced by a bacteria infected with a bacteriophage specific for the bacteria, further comprising at least one holin enzyme, and an appropriate carrier.

2. Therapeutic agent according to claim 1, wherein the shuffled and chimeric lytic enzymes are produced either enzymatically or through recombinant DNA means.

3. Therapeutic agent according to claim 2, wherein the shuffled and chimeric lytic enzymes are produced through recombinant DNA means.

4. Therapeutic agent according to any of claims 1 to 3, wherein the at least one holin enzyme is a shuffled enzyme.

5. Therapeutic agent according to any of claims 1 to 3, wherein the at least one holin enzyme is a chimeric enzyme.

6. Therapeutic agent according to any of claims 1 to 5, wherein the bacteria is Pseudomonas.

7. Therapeutic agent according to any of claims 1 to 5, wherein the bacteria is *Streptococcus.*

8. Therapeutic agent according to any of claims 1 to 5, wherein the bacteria is *Staphylococcus.*

9. Therapeutic agent according to any of claims 1 to 5, wherein the bacteria is *Clostridium*.

10. Therapeutic agent according to any of claims 1 to 9, wherein the at least one chimeric lytic enzyme is Gene EL, a chimeric lysin constructed from bacteriophages phi X174 and MS2 lysis proteins E and L.

11. Therapeutic agent according to any of claims 1 to 9, wherein the at least one chimeric lytic enzyme has been constructed by fusing the region coding for the N-terminal half of the lactococcal phage Tuc2009 lysin and the region coding for the C-terminal domain of the major pneumococcal autolysin.

12. Therapeutic agent according to any of claims 1 to 11, further comprising the enzyme lysostaphin.

## Patentansprüche

1. Therapeutisches Mittel, umfassend eine effektive Menge an mindestens einem lytischen Enzym, an mindestens einem chimären ("chimeric") lytischen Enzym und an mindestens einem mehrfach verteilten ("shuffled") lytischen Enzym, hergestellt von einer Bakterie, die mit einem für diese Bakterie spezifischen Bakteriophagen infiziert ist, weiterhin umfassend mindestens ein Holm-Enzym ("holin enzyme") und einen geeigneten Träger.

2. Therapeutisches Mittel nach Anspruch 1, worin die mehrfach verteilten ("shuffled") lytischen Enzyme und chimären ("chimeric") lytischen Enzyme entweder enzymatisch oder durch rekombinante DNS-Mittel hergestellt sind.

3. Therapeutisches Mittel nach Anspruch 2, worin die mehrfach verteilten ("shuffled") lytischen Enzyme und chimären ("chimeric") lytischen Enzyme durch rekombinante DNS-Mittel hergestellt sind.

4. Therapeutisches Mittel nach einem der Ansprüche 1 bis 3, worin das mindestens eine Holm-Enzym ein mehrfach verteiltes ("shuffled") lytisches Enzym ist.

5. Therapeutisches Mittel nach einem der Ansprüche 1 bis 3, worin das mindestens eine Holin-Enzym ein chimäres ("chimeric") Enzym ist.

6. Therapeutisches Mittel nach einem der Ansprüche 1 bis 5, worin die Bakterie *Pseudomonas* ist.

7. Therapeutisches Mittel nach einem der Ansprüche 1 bis 5, worin die Bakterie *Streptococcus* ist.

8. Therapeutisches Mittel nach einem der Ansprüche 1 bis 5, worin die Bakterie *Staphylococcus* ist.

9. Therapeutisches Mittel nach einem der Ansprüche 1 bis 5, worin die Bakterie *Clostridium* ist.

10. Therapeutisches Mittel nach einem der Ansprüche 1 bis 9, worin das mindestens eine chimäre lytische Enzym Gen E-L ("Gene E-L") ist, ein chimäres Lysin, welches aus den Lysis-Proteinen E und L der Bacteriophagen phi X174 und MS2 aufgebaut wurde.

11. Therapeutisches Mittel nach einem der Ansprüche 1 bis 9, worin das mindestens eine chimäre lytische Enzym aufgebaut wurde durch das Fusionieren der Region, die für die N-terminale Hälfte des Lysin des lactococcalen ("lactococcal") Phagen Tuc2009 kodiert und der Region, die für die C-terminale Domäne des hauptsächlich pneumococcalen ("pneumococcal") Autolysin kodiert.

12. Therapeutisches Mittel nach einem der Ansprüche 1 bis 11, weiterhin umfassend das Enzym Lysostaphin.

## Revendications

1. Agent thérapeutique comprenant un nombre effectif d'au moins un enzyme lytique, au moins un enzyme lytique chimère et au moins un enzyme lytique brouillé (shuffled) produits par une bactérie infectée par un bactériophage spécifique de la bactérie et de plus comprenant au moins un enzyme holine (holin enzyme) et un transporteur approprié.

2. Agent thérapeutique selon la revendication 1, dans lequel les enzymes brouillé (shuffled) et lytique chimère sont produits soit enzymatiquement, soit au moyen d'ADN recombiné.

3. Agent thérapeutique selon la revendication 2, dans lequel les enzymes brouillé (shuffled) et lytique chimère sont produits au moyen d'ADN recombiné.

4. Agent thérapeutique selon une quelconque des revendications 1 à 3, dans lequel ledit au moins un enzyme holine est un enzyme brouillé (shuffled).

5. Agent thérapeutique selon une quelconque des revendications 1 à 3, dans lequel ledit au moins un enzyme holine est un enzyme chimère.

6. Agent thérapeutique selon une quelconque des revendications 1 à 5, dans lequel la bactérie est *Pseudomonas.*

7. Agent thérapeutique selon une quelconque des revendications 1 à 5, dans lequel la bactérie est *Streptococcus*.

8. Agent thérapeutique selon une quelconque des revendications 1 à 5, dans lequel la bactérie est *Staphylococcus.*

9. Agent thérapeutique selon une quelconque des revendications 1 à 5, dans lequel la bactérie est *Clostridium.*

10. Agent thérapeutique selon une quelconque des revendications 1 à 9, dans lequel ledit au moins un enzyme lytique chimère est le gène E-L, une lysine chimère élaborée à partir des protéines de lyse E et L des bactériophages phi X174 et MS2.

11. Agent thérapeutique selon une quelconque des revendications 1 à 9, dans lequel ledit au moins un enzyme lytique chimère a été élaboré en fondant la région codant pour la moitié N-terminale de la lysine du phage lactocoque Tuc2009 et la région codant pour le domaine C-terminal de l'autolysine pneumococcique majeure.

12. Agent thérapeutique selon une quelconque des revendications 1 à 11, comprenant en outre l'enzyme lysostaphine.
